# FASCICULE DE BREVET EUROPEEN

(11) **EP 0 906 750 B1**
(45) Date de publication et mention de la délivrance du brevet: **23.06.2004**
(21) Numéro de dépôt: 98401787.1
(22) Date de dépôt: 16.07.1998
(51) Int. Cl.: A61F 2/04, A61F 2/06

(54) **Prothèse urétrale**
Harnröhrenprothese
Urethral prosthesis

(30) Priorité: 27.08.1997 FR 9710694
(43) Date de publication de la demande: 07.04.1999
(73) Titulaire: PORGES, 92350 Le Plessis-Robinson (FR)
(72) Inventeur: Torchio, Gérard, 91370 Verrieres le Buisson (FR)
(74) Mandataire: Bonnetat, Christian

(56) Documents cités:
- EP-A- 0 631 762
- WO-A-90/04982
- DE-B- 1 250 058
- US-A- 5 064 435
- US-A- 5 197 978
- US-A- 5 354 309

## Description

La présente invention concerne une prothèse destinée à être introduite et maintenue en place dans l'urètre d'un patient de sexe masculin.

On sait que certains patients, par exemple ceux qui sont paraplégiques à la suite d'une rupture de la colonne vertébrale entre la deuxième et la quatrième vertèbre lombaire, sont sujets à des hyperpressions vésicales pré-et per-mictionnelles intempestives. Actuellement, de tels patients doivent porter en permanence une sonde débouchant dans une poche de collecte d'urine extérieure ou bien pratiquer de douloureux sondages plusieurs fois par jour (de l'ordre de six fois quotidiennement), de tels sondages entraînant de plus des risques d'infection. Ces patients sont par ailleurs obligés d'absorber des médicaments anti-cholinergiques pour soulager les douleurs dues aux spasmes vésicaux. Cependant, de tels médicaments engendrent des effets secondaires (manque de salivation, mains moites, ...) et perdent leur efficacité au cours du temps.

La présente invention a pour objet une prothèse susceptible de remédier à ces inconvénients.

A cette fin, selon l'invention, la prothèse urétrale, destinée à un patient de sexe masculin et constituée d'un corps comportant :
- une première partie creuse destinée à être logée dans l'urètre, du côté aval du sphincter, et à maintenir l'urètre ouvert;
- une deuxième partie creuse destinée à être disposée en amont du sphincter, dans l'urètre prostatique, et à assurer l'immobilisation de la prothèse dans l'urètre ; et
- une troisième partie reliant lesdites première et deuxième parties et traversant le sphincter lorsque ladite prothèse est en place dans l'urètre,
   est remarquable en ce que ladite troisième partie est apte, lorsque ladite prothèse est en place dans l'urètre, à empêcher le sphincter de se fermer.

Ainsi, les patients portant une telle prothèse :
- soit sont rendus volontairement incontinents, de sorte qu'ils ne subissent plus les douleurs dues aux spasmes vésicaux et évitent les sondes permanentes ou les autosondages. Pour leur confort, il suffit alors de les munir de moyens susceptibles de collecter ou absorber l'urine qui s'écoule par l'urètre, comme par exemple des serviettes absorbantes;
- soit restent continents par simple résistance urétrale, avec diminution du tonus vésical induite par une modification des réflexes médullaires, de sorte que les douleurs disparaissent également.

On remarquera que, par exemple par le document EP-A-0 631 762, on connaît déjà une prothèse urétrale comportant des première et deuxième parties semblables à celles décrites ci-dessus, et une troisième partie reliant lesdites première et deuxième parties et traversant le sphincter, lorsque la prothèse se trouve en place dans l'urètre. Cependant, dans ce cas, ladite troisième partie est volontairement constituée par un simple fil, afin de ne pas empêcher la fermeture du sphincter.

Dans la prothèse conforme à la présente invention, ladite troisième partie est un élément creux, tubulaire, et son diamètre est choisi pour que l'écoulement de l'urine soit libre au niveau du sphincter, celui-ci s'appuyant contre la paroi externe dudit élément creux.

Afin de pouvoir s'ancrer fermement dans l'urètre prostatique et assurer ainsi l'immobilisation de la prothèse, ladite deuxième partie présente la forme d'un tronc de cône se raccordant par sa petite base audit élément tubulaire de ladite troisième partie.

Ladite première partie présente également la forme d'un tronc de cône se raccordant par sa petite base audit élément tubulaire de ladite troisième partie.

Ainsi, ladite prothèse présente la forme d'un diabolo, dont la partie centrale rétrécie est formée par ladite troisième partie tubulaire. On remarquera que cette partie centrale peut avoir une hauteur relativement faible, correspondant approximativement à l'épaisseur du sphincter dans lequel elle est logée (par exemple de l'ordre de 2 mm), alors que lesdites première et deuxième parties doivent avoir des longueurs plus grandes afin d'assurer leurs fonctions respectives d'ouverture de l'urètre en aval du sphincter et d'ancrage dans l'urètre prostatique.

D'ailleurs, en raison de ces fonctions différentes desdites première et deuxième parties, il est avantageux que le tronc de cône de ladite deuxième partie ait un angle au sommet plus grand que celui du tronc de cône de ladite première partie.

Dans un mode de réalisation avantageux, lesdites première, deuxième et troisième parties sont parties intégrantes les unes des autres, de sorte que ladite prothèse est formée d'une unique pièce. De préférence, cette unique pièce est formée par un enroulement de fil élastique, par exemple du fil d'acier inoxydable de qualité médicale, à spires jointives.

La prothèse conforme à la présente invention peut se présenter sous la forme d'une pièce de révolution, c'est-à-dire à section transversale circulaire. Cependant, il peut en résulter des difficultés de mise en place dans l'urètre, notamment à cause de la forme tronconique évasée de ladite deuxième partie, qui doit traverser le sphincter pour venir se loger dans l'urètre prostatique. Aussi est-il avantageux de prévoir au moins ladite deuxième partie élastiquement déformable pour pouvoir prendre un premier état sous contrainte pour lequel sa section est au plus égale à celle de l'urètre et un second état libre pour lequel sa section est telle qu'elle peut prendre appui sur la paroi interne de l'urètre pour immobiliser ladite prothèse dans l'urètre.

Pour améliorer encore la mise en place de la prothèse dans l'urètre, ladite deuxième partie peut de plus présenter la forme au moins approximative d'un tronc de cône aplati à section oblongue, la petite dimension de ladite section étant constante et égale au diamètre dudit élément tubulaire creux de ladite troisième partie. Il est alors avantageux que ladite première partie présente également la forme au moins approximative d'un tronc de cône aplati à section oblongue, la petite dimension de ladite section étant constante et égale au diamètre dudit élément tubulaire creux de ladite troisième partie. Ainsi, la prothèse conforme à la présente invention présente la forme approximative d'un diabolo aplati, dont la petite dimension de la section est constante et égale au diamètre dudit élément tubulaire creux de ladite troisième partie.

Par suite, une telle prothèse conforme à l'invention peut être radialement comprimée pour être logée dans un tube d'introduction pouvant facilement être introduit dans l'urètre. Lorsque le tube d'introduction a atteint l'emplacement approprié pour la prothèse, celle-ci est éjectée par l'extrémité distale du tube, par exemple grâce à un poussoir, et elle reprend spontanément et élastiquement sa forme en tronc de cône aplati.

Les figures du dessin annexé feront bien comprendre comment l'invention peut être réalisée. Sur ces figures, des références identiques désignent des éléments semblables.
La figure 1 est une vue en élévation d'un premier mode de réalisation de la prothèse urétrale conforme à la présente invention.
La figure 2 est une vue en plan de la prothèse de la figure 1.
La figure 3 est une vue schématique de la prothèse de l'invention, mise en place dans un urètre.
Les figures 4 et 5 sont deux vues en élévation, selon deux directions perpendiculaires, d'un second mode de réalisation de la prothèse selon l'invention.
La figure 6 est une vue en plan de la prothèse des figures 4 et 5.
La figure 7 montre la prothèse urétrale des figures 4, 5 et 6 insérée dans un tube de mise en place.
La figure 8 correspond à la figure 7, ladite prothèse urétrale étant partiellement poussée hors dudit tube de mise en place.

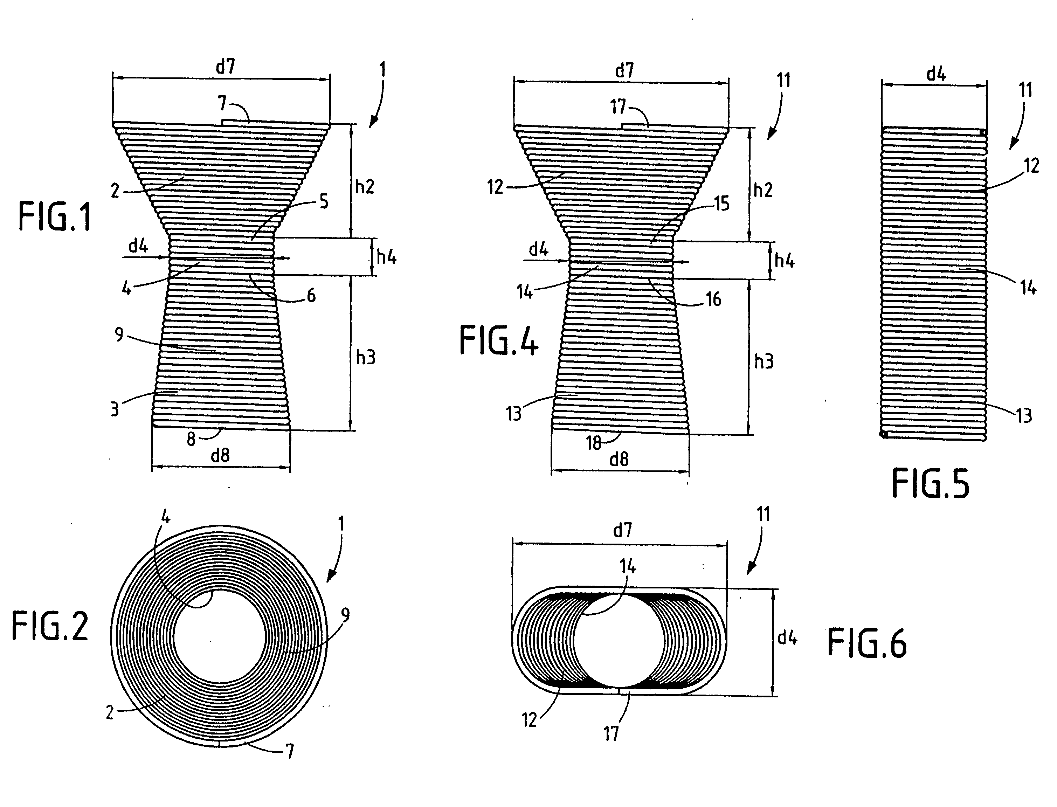

La prothèse urétrale 1, conforme à la présente invention et montrée par les figures 1 et 2, présente la forme d'un diabolo comportant deux troncs de cône creux opposés 2 et 3, raccordés l'un à l'autre par une portion tubulaire de rétrécissement 4. Les troncs de cône 2 et 3 sont à sections circulaires et leurs petites bases 5 et 6, respectivement raccordées l'une à l'autre par la portion tubulaire 4, présentent le même diamètre d4 que celle-ci. En revanche, le diamètre d7 de la grande base 7 du tronc de cône 2 est supérieur au diamètre d8 de la grande base 8 du tronc de cône 3, alors que la hauteur h2 du tronc de cône 2 est plus petite que la hauteur h3 du tronc de cône 3. Il en résulte que le tronc de cône 3 a un angle au sommet plus petit que celui du tronc de cône 2.

Dans un exemple de réalisation particulier (non limitatif), les différentes dimensions mentionnées ci-dessus sont les suivantes :
- pour la portion tubulaire de rétrécissement 4, hauteur h4 égale à 2 mm et diamètre d4 égal à 5,3 mm ;
- pour le tronc de cône 2, hauteur h2 égale à 6 mm, diamètre d4 de la petite base 5 égal à 5,3 mm et diamètre d7 de la grande base 7 égal à 12 mm ; et
- pour le tronc de cône 3, hauteur h3 égale à 8 mm, diamètre d4 de la petite base 6 égal à 5,3 mm et diamètre d8 de la grande base 8 égal à 8 mm.

On constate donc que la hauteur totale h2+h4+h3 de la prothèse est égale à 16 mm et que la hauteur h4 de la portion tubulaire 4 ne représente que 1/8 de cette hauteur totale.

Comme cela est illustré sur les figures 1 et 2, la prothèse 1 est constituée d'une seule pièce réalisée par enroulement à spires jointives (par exemple sur un mandrin approprié non représenté) d'un fil élastique 9, par exemple en acier inoxydable de quantité médicale, ce fil métallique étant continu depuis la grande base 7 du tronc de cône 2 jusqu'à la grande base 8 du tronc de cône 3.

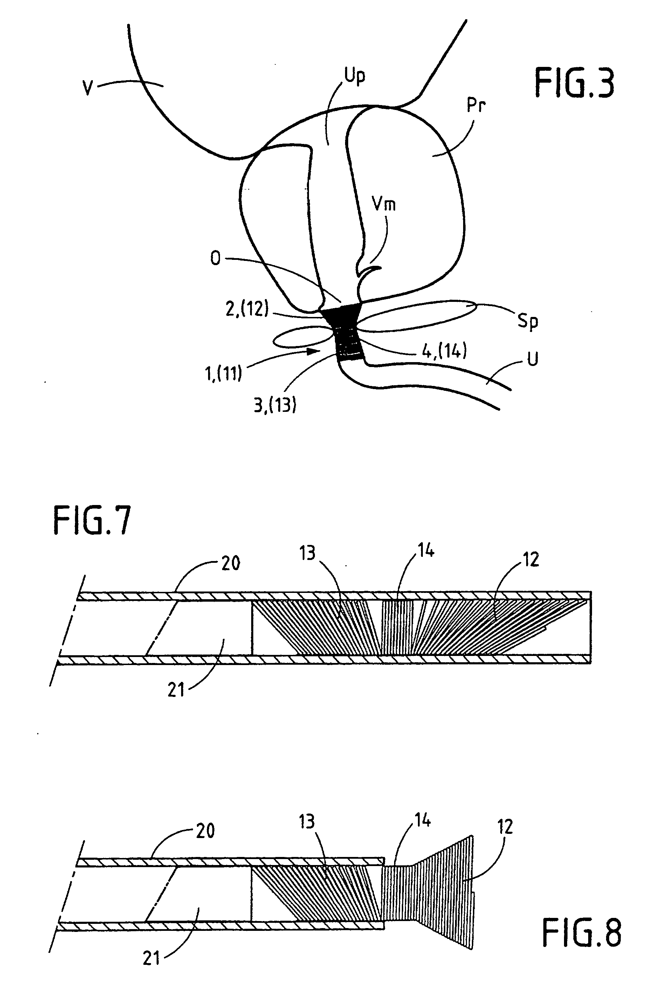

Sur la figure 3, on a représenté la prothèse 1 en place dans l'urètre. Cette figure 3 montre schématiquement la vessie V, l'urètre U, le sphincter Sp, l'urètre prostatique Up, la prostate Pr et le vérumontanum Vm. Comme on peut le voir, la prothèse 1 est disposée de façon que sa portion tubulaire 4 se trouve au niveau du sphincter, pour l'écarter et l'empêcher de se fermer, le tronc de cône 3 se trouvant dans l'urètre du côté aval du sphincter et maintenant ledit urètre ouvert, tandis que le tronc de cône 2 est ancré, de par sa forme conique, dans l'urètre prostatique Up, au voisinage de l'ouverture O par laquelle l'urètre débouche dans l'urètre prostatique Up.

Dans la variante de réalisation des figures 4, 5 et 6, la prothèse urétrale 11, également réalisée par un enroulement à spires jointives d'un fil élastique, est constituée de trois parties 12, 13 et 14 correspondant respectivement aux parties 2, 3 et 4 de la prothèse 1. Cette prothèse 11 présente la forme d'un diabolo aplati, les parties 12 et 13 n'ayant plus une section circulaire comme les parties 2 et 3, mais des sections oblongues. Les petites dimensions des sections des parties 12 et 13 sont égales entre elles et constantes, en étant égales au diamètre d4 de la troisième partie 14. Ainsi, comme le montrent les figures 5 et 6, la prothèse 11 a une épaisseur constante égale à d4. En revanche, les grandes dimensions des sections des parties 12 et 13 varient comme les diamètres des sections des parties 2 et 3. Ainsi, la grande dimension de la section de la partie 12 varie de d4, au niveau de sa petite base 15, jusqu'à d7, au niveau de la grande base 17. De même, la grande dimension de la section de la partie 13 varie de d4, au niveau de la petite base 16, jusqu'à d8, au niveau de la grande base 18.

Dans l'exemple représenté sur la figure 4, les parties 12, 13 et 14 ont respectivement la même hauteur h2, h3, h4 que les parties 2, 3 et 4 de la prothèse 1.

On voit ainsi que, grâce à sa forme aplatie et à son élasticité due à l'enroulement de fil élastique, la prothèse 11 peut aisément être introduite, en étant radialement comprimée de façon élastique, dans un tube 20, dont le diamètre lui permet d'être aisément introduit dans l'urètre U (voir la figure 7). Le tube 20 étant introduit dans l'urètre jusqu'au sphincter Sp, il est possible d'éjecter la prothèse 11 par l'extrémité distale du tube 20, par exemple grâce à un poussoir 21, intérieur audit tube. En sortant du tube 20, la prothèse 11 reprend élastiquement sa forme en diabolo aplati, de façon spontanée, comme cela est illustré schématiquement par la figure 8.

On voit donc que la mise en place de la prothèse 11 dans l'urètre, de façon que sa troisième partie 14 se trouve dans le sphincter Sp, est particulièrement simple.

Le retrait des prothèses 1 et 11 peut être effectué en débobinant lesdites prothèses.

## Revendications

1. Prothèse urétrale (1, 11), destinée à un patient de sexe masculin et apte, lorsque ladite prothèse est en place dans l'urètre, à empêcher le sphincter de se fermer, constituée par un corps creux comportant :
- une première partie creuse (3, 13) destinée à être logée dans l'urètre (U), du côté aval du sphincter (Sp), et à maintenir l'urètre ouvert ;
- une deuxième partie creuse (2, 12) destinée à être disposée en amont du sphincter (Sp), dans l'urètre prostatique (Up) ; et
- une troisième partie (4, 14) constituée par un élément creux reliant lesdites première et deuxième parties et traversant le sphincter lorsque ladite prothèse est en place dans l'urètre,
**caractérisée en ce que** :
- ladite troisième partie (4, 14) est un élément tubulaire ;
- ladite deuxième partie (2, 12) présente la forme d'un tronc de cône se raccordant par sa petite base (5, 15) audit élément tubulaire de ladite troisième partie (4,14), de façon à assurer l'immobilisation de ladite prothèse dans l'urètre ; et
- ladite première partie (3, 13) présente la forme d'un tronc de cône se raccordant par sa petite base (6, 16) audit élément tubulaire de ladite troisième partie (4, 14).

2. Prothèse urétrale selon la revendication 1,
**caractérisée en ce que** le tronc de cône de ladite deuxième partie (2, 12) a un angle au sommet plus grand que celui du tronc de cône de ladite première partie (4, 14).

3. Prothèse urétrale selon l'une des revendications 1 ou 2,
**caractérisée en ce qu'**au moins ladite deuxième partie est élastiquement déformable pour pouvoir prendre un premier état sous contrainte pour lequel sa section est au plus égale à celle de l'urètre et un second état libre pour lequel sa section est telle qu'elle peut prendre appui sur la paroi interne de l'urètre prostatique pour immobiliser ladite prothèse dans l'urètre.

4. Prothèse urétrale selon l'une des revendications 1 à 3,
**caractérisée en ce que** lesdites première, deuxième et troisième parties sont parties intégrantes les unes des autres, de sorte que ladite prothèse est formée d'une unique pièce (1, 11).

5. Prothèse urétrale selon la revendication 4,
**caractérisée en ce que** ladite pièce (1, 21) est formée par un enroulement de fil élastique, à spires jointives.

6. Prothèse urétrale selon l'une des revendications 3 à 5,
**caractérisée en ce que** ladite deuxième partie (12) présente la forme au moins approximative d'un tronc de cône aplati à section oblongue, la petite dimension de ladite section étant constante et égale au diamètre dudit élément tubulaire creux (14) de ladite troisième partie.

7. Prothèse urétrale selon l'une des revendications 3 à 6,
**caractérisée en ce que** ladite première partie (13) présente la forme au moins approximative d'un tronc de cône aplati à section oblongue, la petite dimension de ladite section étant constante et égale au diamètre dudit élément tubulaire creux (14) de ladite troisième partie.

8. Prothèse urétrale selon l'une des revendications 6 ou 7,
**caractérisée en ce qu'**elle présente la forme approximative d'un diabolo aplati, dont la petite dimension de la section est constante et égale au diamètre dudit élément tubulaire creux (14) de ladite troisième partie.

## Patentansprüche

1. Harnröhrenprothese (1, 11), welche für einen Patienten männlichen Geschlechts bestimmt ist und welche geeignet ist, wenn sich die Prothese in der Harnröhre an Ort und Stelle befindet, zu verhindern, dass sich der Schließmuskel schließt, und welche aus einem Hohlkörper gebildet wird, der umfasst:
- ein erstes hohles Teil (3, 13), welches dazu bestimmt ist, in der Harnröhre (U) auf der Seite unterhalb des Schließmuskels (Sp) untergebracht zu werden und die Harnröhre offen zu halten;
- ein zweites hohles Teil (2, 12), welches dazu bestimmt ist, oberhalb des Schließmuskels (Sp) in der Pars prostatica urethrae (Up) angeordnet zu werden; und
- ein drittes Teil (4, 14), welches aus einem hohlen Element besteht, welches das erste Teil und das zweite Teil verbindet und durch den Schließmuskel hindurch führt, wenn sich die Prothese in der Harnröhre an Ort und Stelle befindet,
**dadurch gekennzeichnet, dass**:
- das dritte Teil (4, 14) ein röhrenförmiges Element ist;
- das zweite Teil (2, 12) die Form eines Kegelstumpfes aufweist, der mit seiner kleinen Grundfläche (5, 15) mit dem röhrenförmigen Element des dritten Teils (4, 14) so verbunden ist, dass der feste Sitz der Prothese in der Harnröhre gewährleistet wird; und
- das erste Teil (3, 13) die Form eines Kegelstumpfes aufweist, der mit seiner kleinen Grundfläche (6, 16) mit dem röhrenförmigen Element des dritten Teils (4, 14) verbunden ist.

2. Harnröhrenprothese nach Anspruch 1,
**dadurch gekennzeichnet, dass** der Kegelstumpf des zweiten Teils (2, 12) einen Kegelwinkel aufweist, der größer als der des Kegelstumpfes des ersten Teils (4, 14) ist.

3. Harnröhrenprothese nach einem der Ansprüche 1 oder 2,
**dadurch gekennzeichnet, dass** zumindest das zweite Teil elastisch verformbar ist, damit es einen ersten Zustand unter Spannung annehmen kann, für welchen sein Querschnitt höchstens gleich demjenigen der Harnröhre ist, und einen zweiten freien Zustand, für den sein Querschnitt dergestalt ist, dass er an der inneren Wandung der Pars prostatica urethrae Halt findet, um die Prothese in der Harnröhre festzuhalten.

4. Harnröhrenprothese nach einem der Ansprüche 1 bis 3,
**dadurch gekennzeichnet, dass** die ersten, zweiten und dritten Teile untereinander fest miteinander verbunden sind, so dass die Prothese aus einem einzigen Stück (1, 11) gebildet ist.

5. Harnröhrenprothese nach Anspruch 4,
**dadurch gekennzeichnet, dass** das Stück (1, 21) aus einer Wicklung aus elastischem Draht mit aneinander anliegenden Windungen gebildet wird.

6. Harnröhrenprothese nach einem der Ansprüche 3 bis 5,
**dadurch gekennzeichnet, dass** das zweite Teil (12) zumindest näherungsweise die Form eines abgeflachten Kegelstumpfes mit länglichem Querschnitt aufweist, wobei die kleine Abmessung des Querschnitts konstant und gleich dem Durchmesser des hohlen röhrenförmigen Elements (14) des dritten Teils ist.

7. Harnröhrenprothese nach einem der Ansprüche 3 bis 6,
**dadurch gekennzeichnet, dass** das erste Teil (13) zumindest näherungsweise die Form eines abgeflachten Kegelstumpfes mit länglichem Querschnitt aufweist, wobei die kleine Abmessung des Querschnitts konstant und gleich dem Durchmesser des hohlen röhrenförmigen Elements (14) des dritten Teils ist.

8. Harnröhrenprothese nach einem der Ansprüche 6 oder 7,
**dadurch gekennzeichnet, dass** sie annähernd die Form einer doppelkegligen Rolle aufweist, deren kleine Abmessung des Querschnitts konstant und gleich dem Durchmesser des hohlen röhrenförmigen Elements (14) des dritten Teils ist.

## Claims

1. Urethral prosthesis (1, 11) intended for a male patient and able to prevent the sphincter from closing when said prosthesis is in place in the urethra, said prosthesis being formed by a hollow body comprising:
- a first hollow part (3, 13) intended to be lodged in the urethra (U), downstream of the sphincter (Sp), and to keep the urethra open;
- a second hollow part (2, 12) intended to be arranged upstream of the sphincter (Sp), in the prostatic urethra (Up); and
- a third part (4, 14) formed by a hollow element connecting said first and second parts and traversing the sphincter when said prosthesis is in place in the urethra,
**characterized in that**:
- said third part (4, 14) is a tubular element;
- said second part (2, 12) has the form of a truncated cone connected via its small base (5, 15) to said tubular element of said third part (4, 14), in such a way as to ensure the immobilization of said prosthesis in the urethra; and
- said first part (3, 13) has the form of a truncated cone connected via its small base (6, 16) to said tubular element of said third part (4, 14).

2. Urethral prosthesis according to Claim 1, **characterized in that** the truncated cone of said second part (2, 12) has an apex angle greater than that of the truncated cone of said first part (4, 14).

3. Urethral prosthesis according to either of Claims 1 and 2, **characterized in that** at least said second part is elastically deformable so as to be able to assume a first, stressed state in which its cross section is at most equal to that of the urethra, and a second, free state in which its cross section is such that it can bear on the inner wall of the prostatic urethra and thus immobilize said prosthesis in the urethra.

4. Urethral prosthesis according to one of Claims 1 to 3, **characterized in that** said first, second and third parts are parts which are integral with one another, so that said prosthesis is formed by a single component (1, 11).

5. Urethral prosthesis according to Claim 4, **characterized in that** said component (1, 21) is formed by winding elastic wire with contiguous turns.

6. Urethral prosthesis according to one of Claims 3 to 5, **characterized in that** said second part (12) has the at least approximate form of a flattened truncated cone of oblong cross section, the small dimension of said cross section being constant and equal to the diameter of said hollow tubular element (14) of said third part.

7. Urethral prosthesis according to one of Claims 3 to 6, **characterized in that** said first part (13) has the at least approximate form of a flattened truncated cone of oblong cross section, the small dimension of said cross section being constant and equal to the diameter of said hollow tubular element (14) of said third part.

8. Urethral prosthesis according to either of Claims 6 and 7, **characterized in that** it has the approximate form of a flattened hourglass, of which the small dimension of the cross section is constant and equal to the diameter of said hollow tubular element (14) of said third part.
